# EUROPÄISCHE PATENTSCHRIFT

(11) **EP 1 937 074 B1**
(45) Veröffentlichungstag und Bekanntmachung des Hinweises auf die Patenterteilung: **21.01.2015**
(21) Anmeldenummer: 06792313.6
(22) Anmeldetag: 28.09.2006
(51) Int. Cl.: A01N 51/00, A01N 53/00, A01N 25/04, A01N 25/30, A01P 7/00

(54) **SUSPENSIONSKONZENTRATE AUF ÖLBASIS**
OIL BASED SUSPENSION CONCENTRATES
CONCENTRES DE SUSPENSION A BASE D'HUILE

(30) Priorität: 11.10.2005 DE 102005048539
(43) Veröffentlichungstag der Anmeldung: 02.07.2008
(73) Patentinhaber: Bayer Intellectual Property GmbH, 40789 Monheim am Rhein (DE)
(72) Erfinder: VERMEER, Ronald, 51371 Leverkusen (DE); EBERHARD, Manuela, 51371 Leverkusen (DE)
(74) Vertreter: BIP Patents
(86) Internationale Anmeldenummer: PCT/EP2006/009433
(87) Internationale Veröffentlichungsnummer: WO 2007/042152

(56) Entgegenhaltungen:
- WO-A-03/000053
- WO-A-2006/111279
- WO-A2-02/098230
- WO-A2-2005/084435
- FR-A1- 2 784 011
- DATABASE WPI Week 200343 Derwent Publications Ltd., London, GB; AN 2003-451684 XP002411920 & JP 2002 370901 A (NIPPON SODA CO) 24. Dezember 2002 (2002-12-24)

## Beschreibung

Die vorliegende Erfindung betrifft neue, ölbasierende Suspensionskonzentrate von agrochemische Wirkstoffen, ein Verfahren zur Herstellung dieser Formulierungen und deren Verwendung zur Applikation der enthaltenen Wirkstoffe.

Systemische agrochemische Wirkstoffen, insbesondere systemische Insektizide, brauchen zur Entfaltung der biologischen Wirkung eine Formulierung, welche es ermöglicht, dass die Wirkstoffe in die Pflanze / den Zielorganismen aufgenommen werden. Üblicherweise werden deshalb systemische agrochemische Wirkstoffen als Emulsionskonzentrat (EC), Soluble Liquid (SL) und/oder ölbasierendes Suspensionskonzentrat (OD) formuliert. In einer EC und SL liegt der Wirkstoff in gelöster Form vor, bei einer OD-Formulierung als Feststoff. Im letzten Fall wird die biologische Wirkung durch Zugabe von Penetrationsförderern ermöglicht. Kontaktwirkstoffe wie zum Beispiel Pyrethroide, werden, vor allem wenn eine hohe Initialwirkung notwendig ist, bevorzugt als EC formuliert. Suspensionskonzentrate (SC) oder Wettable Granules (WG) sind meistens technisch möglich, zeigen aber nicht die geforderte Initialwirkung.

Mischformulierungen von systemischen und Kontaktinsektiziden wie zum Beispiel eine Mischung von Imidacloprid mit Beta-Cyfluthrin, sind von hohem Interesse als Alternative für die sehr akuttoxischen Organophosphate. Solche Mischungen kommen als Alternative für die Anwendung von Organophosphaten nur in Frage, wenn eine hohe Initialwirkung vorhanden ist und Formulierungen mit einem hohen Wirkstoffgehalt vorliegen. Es sind keine EC-Formulierungen mit hohen Gehalten an sowohl Imidacloprid als auch Beta-Cyfluthrin bekannt, weil es kein Lösemittel gibt welches beide Wirkstoffe in geeigneten Menge lösen kann. Deshalb kommen nur ölbasierende oder wasser-freie Suspensionskonzentrate in Frage.

Es sind bereits zahlreiche wasserfreie Suspensionskonzentrate von agrochemischen Wirkstoffen bekannt geworden. So werden in der EP-A 0 789 999 Formulierungen dieses Typs beschrieben, die neben Wirkstoff und Öl ein Gemisch verschiedener Tenside - darunter auch solche, die als Penetrationsförderer dienen - sowie ein hydrophobiertes Alumoschichtsilikat als Verdickungsmittel enthalten. Im genannte Patent sind als geeignete Wirkstoffe beschrieben, die die eine Löslichkeit in Öl kleiner als 5 g/l, bevorzugt kleiner als I g/l insbesondere kleiner als 0,1 g/l zeigen.

Weiterhin sind aus der US-A 6 165 940 schon nicht-wässrige Suspensionskonzentrate bekannt, in denen außer agrochemischem Wirkstoff, Penetrationsförderer und Tensid bzw. Tensid-Gemisch ein organisches Solvens vorhanden ist, wobei als derartige Lösungsmittel auch Paraffinöl oder Pflanzenöl-Ester in Frage kommen. Diese Erfindung beschreibt Suspensionskonzentrate bestehende aus festen Wirkstoff(en) und organische Lösemittel, wobei der Wirkstoff weniger als gemäßigt löslich ist. Eine Löslichkeit von weniger als 10 g/l, bevorzugt weniger als 5 g/l ist explizit genannt.

DE-A 10 129 855 beschreibt weitere Suspensionskonzentrate auf Ölbasis, die agrochemische Wirkstoffe, Penetrationsförderer und Tenside enthalten.

Nachteil der oben genannten Formulierungen ist es, dass es nicht möglich ist, einen schlecht löslichen Wirkstoff (weniger als 10 g/l) in Kombination mit einem gemäßigt löslichen Wirkstoff (10 bis 50 g/l bei Raumtemperatur) als stabile ölbasierende Suspensionskonzentrat zu entwickeln, ohne dass Kristallwachstum nach Lagerung auftretet. Das Wachsen der Wirkstoff Kristalle in einer Formulierung ist ein erheblicher Nachteil für den Anwender, weil dadurch die Siebe seiner Spritzanlage bei der Anwendung des Produkts verstopft werden können.

Die vorliegende Erfindung hat als Ziel stabile, lagerbare, ölbasierende Suspensionskonzentrate bestehend aus einem schlecht löslichen Wirkstoff und einem gemäßigt löslichen Wirkstoff, welcher in höherer Konzentration als die Löslichkeitsgrenze in der Formulierung enthalten ist, zu entwickeln.

Es wurden nun neue Suspensionskonzentrate auf Ölbasis gefunden, enthaltend
- mindestens einen bei Raumtemperatur festen Wirkstoff aus der Reihe der Neonikotinoide,
- mindestens einen bei Raumtemperatur festen Wirkstoff aus der Reihe der Pyrethroide,
- mindestens einen Penetrationsförderer.
- mindestens ein Pflanzenöl,
- Cyclohexanon
- mindestens ein nicht-ionisches Tensid und/oder mindestens ein anionisches Tensid und
- einen oder mehrere Zusatzstoffen aus den Gruppen der Emulgiermittel, der schaumhemmenden Mittel, der Konservierungsmittel, der Antioxydantien, der Spreitmittel, der Farbstoffe und/oder einen Verdicker.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die nachher und in der Literatur (Baur et al., 1997, Pesticide Science 51, 131-152) beschriebene Methode kann zur Bestimmung dieser Eigenschaft eingesetzt werden.

Weiterhin wurde gefunden, dass sich die erfindungsgemäßen Suspensionskonzentrate auf Ölbasis herstellen lassen, indem man
- mindestens einen bei Raumtemperatur festen Wirkstoff aus der Reihe der Neonikotinoide,
- mindestens einen bei Raumtemperatur festen Wirkstoff aus der Reihe der Pyrethroide,
- mindestens einen Penetrationsförderer,
- mindestens ein Pflanzenöl,
- Cyclohexanon
- mindestens ein nicht-ionisches Tensid und/oder mindestens ein anionischen Tensid und
- einen oder mehrere Zusatzstoffe aus den Gruppen der Emulgiermittel, der schaumhemmenden Mittel, der Konservierungsmittel, der Antioxydantien, der Spreitmittel, der Farbstoffe und/oder einen Verdicker
miteinander vermischt und die entstehende Suspension gegebenenfalls anschließend mahlt.

Schließlich wurde gefunden, dass sich die erfindungsgemäßen Suspensionskonzentrate auf Ölbasis sehr gut zur Applikation der enthaltenen agrochemischen Wirkstoffe auf Pflanzen und/oder deren Lebensraum eignen.

Es ist als äußerst überraschend zu bezeichnen, dass die erfindungsgemäßen Suspensionskonzentrate auf Ölbasis eine sehr gute Stabilität aufweisen, und insbesondere dass auch nach Lagerung bei wechselnder Temperatur kein signifikantes Kristallwachstum beobachtet wurde. Unerwartet ist auch, dass sie eine deutlich bessere biologische Wirksamkeit zeigen als die am ähnlichsten zusammengesetzten, vorbenannten Formulierungen. Insbesondere ist es unerwartet, dass eine sehr hohe Initialwirkung des Kontaktwirkstoffes gefunden wird obwohl dieser Wirkstoff teilweise als Feststoff vorliegt.

Als Wirkstoffe kommen Insektizide aus der Reihe der Neonikotinoide in Frage. Diese eignen sich hervorragend zur Bekämpfung tierischer Schädlinge. Insektizide aus der Reihe der Neonikotinoide lassen sich durch folgende Formel (II) 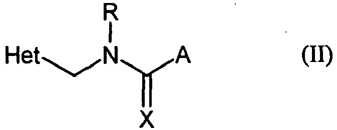 worin
- Het: für einen Heterocyclus ausgewählt au der folgenden Gruppe von Heterocyclen steht: 2-Chlorpyrid-5-yl, 2-Methylpyrid-5-yl, 1-Oxido-3-pyridino, 2-Chlor-1-oxido-5-pyridino, 2,3-Dichlor-1-oxido-5-pyridino, Tetrahydrofuran-3-yl, 5-Methyl-tetrahydrofuran-3yl, 2-Chlorothiazol-5-yl,
- A: für N(R¹)(R²) oder S(R²) steht,
worin
R¹ für Wasserstoff, C₁-C₆-Alkyl, Phenyl-C₁-C₄-Alkyl, C₃-C₆-Cycloalkyl, C₂-C₆-Alkenyl oder C₂-C₆-Alkinyl steht, und
R² für C₁-C₆-Alkyl, C₂-C₆-Alkenyl, C₂-C₆-Alkinyl -C(=O)-CH₃ oder Benzyl steht,
- R: für C₁-C₆-Alkyl, C₂-C₆Alkenyl, C₂-C₆-Alkinyl -C(=O)-CH₃ oder Benzyl steht oder gemeinsam mit R² für eine der folgenden Gruppen steht:
-CH₂-CH₂-, -CH₂-CH₂-CH₂-, -CH₂-O-CH₂-, -CH₂-S-CH₂-, -CH₂-NH-CH₂-, -CH₂-N-(CH₃)-CH₂- und
- X: für N-NO₂, N-CN oder CH-NO₂ steht,
beschreiben (siehe z.B. EP-A1-192 606, EP-A2-580 533, EP-A2-376 279, EP-A2-235 725).

Im Einzelnen seien folgende Verbindungen genannt, die erfindungsgemäß verwendet werden können.

Eine erfindungsgemäß bevorzugt verwendete Verbindung ist Thiamethoxam.

Thiamethoxam besitzt die Formel 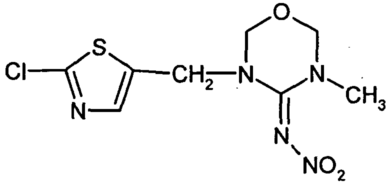 und ist bekannt aus EP A2 0 580 533.

Eine weitere erfindungsgemäß bevorzugt verwendete Verbindung ist Clothianidin.

Clothianidin besitzt die Formel 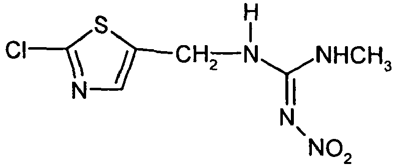 und ist bekannt aus EP A2 0 376 279.

Eine weitere erfindungsgemäß bevorzugt verwendete Verbindung ist Thiacloprid.

Thiacloprid besitzt die Formel 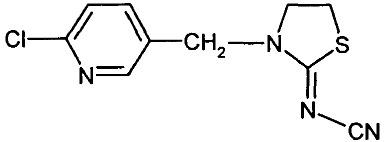 und ist bekannt aus EP A2 0 235 725.

Eine weitere erfindungsgemäß bevorzugt verwendete Verbindung ist Dinotefuran.

Dinotefuran besitzt die Formel 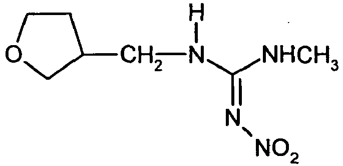 und ist bekannt aus EP A1 0 649 845.

Eine weitere erfindungsgemäß bevorzugt verwendete Verbindung ist Acetamiprid.

Acetamiprid besitzt die Formel 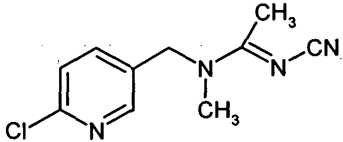 und ist bekannt aus WO A1 91/04965.

Eine weitere erfindungsgemäß bevorzugt verwendete Verbindung ist Nitenpyram.

Nitenpyram besitzt die Formel 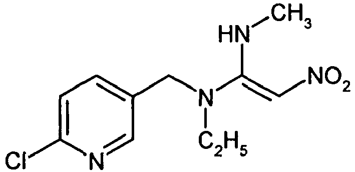 und ist bekannt aus EP A2 0 302 389.

Eine weitere erfindungsgemäß bevorzugt verwendete Verbindung ist Imidacloprid.

Imidacloprid besitzt die Formel 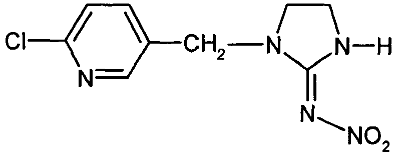 und ist bekannt aus EP 0 192 060.

Besonders bevorzugt ist Imidacloprid.

Als weitere Wirkstoffe kommen solche aus der Gruppe der Pyrethroide in Frage, z.B. Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (-1R- isomer), Tralomethrin, Transfluthrin, ZXI 8901, Pyrethrins (pyrethrum). Bevorzugt werden beta-Cyfluthrin und Deltamethrin.

Bevorzugte Penetrationsförderer sind Alkanol-alkoxylate der Formel

R-O-(-AO)ₘR' (I)

in welcher
- R: für geradkettiges oder verzweigtes Alkyl mit 4 bis 20 Kohlenstoffatomen steht,
- R': für H, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- m: für Zahlen von 2 bis 30 steht.

Eine besonders bevorzugte Gruppe von Penetrationsförderern sind Alkanolalkoxylate der Formel

R-O-(-EO-)ₙ-R' (I-a)

in welcher
- R: die oben angegebene Bedeutung hat,
- R': die oben angegebene Bedeutung hat,
- EO: für -CH₂-CH₂-O-steht und
- n: für Zahlen von 2 bis 20 steht.

Eine weitere besonders bevorzugte Gruppe von Penetrationsförderern sind Alkanol-alkoxylate der Formel

R-O-(-EO-)ₚ(-PO-)_{q}-R' (I-b)

in welcher
- R: die oben angegebene Bedeutung hat,
- R': die oben angegebene Bedeutung hat,
- EO: für -CH₂-CH₂-O- steht,
- PO: für 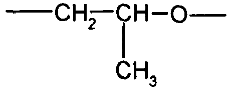 steht,
- p: für Zahlen von 1 bis 10 steht und
- q: für Zahlen von 1 bis 10 steht.

Eine weitere besonders bevorzugte Gruppe von Penetrationsförderern sind Alkanol-Alkoxylate der Formel

R-O-(-PO-)ᵣ-(EO-)ₛ-R' (I-c)

in welcher
- R: die oben angegebene Bedeutung hat,
- R': die oben angegebene Bedeutung hat,
- EO: für -CH₂-CH₂-O- steht,
- PO: für 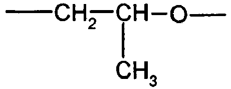 steht,
- r: für Zahlen von 1 bis 10 steht und
- s: für Zahlen von 1 bis 10 steht.

Eine weitere besonders bevorzugte Gruppe von Penetrationsförderern sind Alkanol-alkoacylate der Formel (I-e)

R-O-(-EO-)ₚ-(-BO-)_{q}-R' (I-d)

in welcher
- R und R': die oben angegebenen Bedeutungen haben,
- EO: für CH₂-CH₂-O- steht,
- BO: für 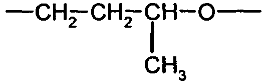 steht,
- p: für Zahlen von 1 bis 10 steht und
- q: für Zahlen von 1 bis 10 steht.

Eine weitere besonders bevorzugte Gruppe von Penetrationsförderern sind Alkanol-alkoxylate der Formel (I-f)

R-O-(-BO-)ᵣ-(-EO-)ₛ-R' (I-e)

in welcher
- R und R': die oben angegebenen Bedeutungen haben,
- BO: für 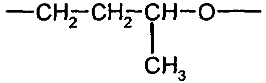 steht,
- EO: für CH₂-CH₂-O- steht,
- r: für Zahlen von 1 bis 10 steht und
- s: für Zahlen von 1 bis 10 steht.

Eine weitere besonders bevorzugte Gruppe von Penetrationsförderern sind Alkanol-Alkoxylate der Formel

CH₃-(CH₂)ₜ-CH₂-O-(-CH₂-CH₂-O-)ᵤ-R' (I-f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmitoyl, Stearyl oder Eicosyl.

Als Beispiel für ein Alkanol-Alkoxylat der Formel (I-c) sei 2-Ethyl-hexyl-alkoxylat der Formel 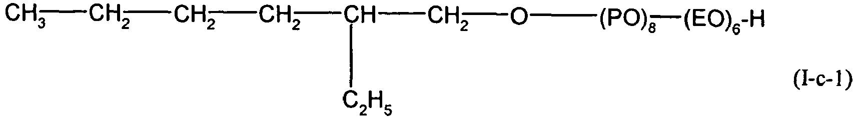 in welcher
- EO: für -CH₂-CH₂-O- steht,
- PO: für 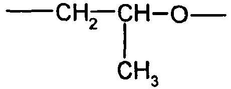 steht und
die Zahlen 8 und 6 Durchschnittswerte darstellen, genannt.

Als Beispiel für ein Alkanol-Alkoxylat der Formel (I-d) sei die Formel

CH₃-(CH₂)₁₀-O-(-EO-)₆-(-BO-)₂-CH₃ (I-d-1)

in welcher
- EO: für CH₂-CH₂-O- steht,
- BO: für 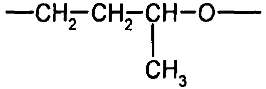 steht und
die Zahlen 10, 6 und 2 Durchschnittswerte darstellen, genannt.

Besonders bevorzugte Alkanol-Alkoxylate der Formel (I-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (I-f-1)

CH₃-(CH₂)ₜ-CH₂-O-(-CH₂-CH₂-O-)ᵤ-R' (I-f-1)

in welcher
- t: für den Durchschnittswert 10,5 steht und
- u: für den Durchschnittswert 8,4 steht.

Ebenfalls ganz besonders bevorzugt genannt sei Alkanol-Alkoxylat der Formel (I-f-1-1)

CH₃-(CH₂)ₜ-CH₂-O-(-CH₂-CH₂-O-)ᵤ-H (I-f-1-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt oder lassen sich nach bekannten Methoden herstellen (vgl. WO 98-35 553, WO 00-35 278 und EP-A 0 681 865).

Als Pflanzenöle kommen alle üblicherweise in agrochemischen Mitteln einsetzbaren, aus Pflanzen gewinnbaren Öle in Frage. Beispielhaft genannt seien Sonnenblumenöl, Rapsöl, Olivenöl, Rizinusöl, Rüböl, Maiskernöl, Baumwollsaatöl und Sojabohnenöl.

Die erfindungsgemäßen Suspensionskonzentrate auf Ölbasis enthalten mindestens ein nicht-ionisches Tensid bzw. Dispergierhilfsmittel und/oder mindestens ein anionisches Tensid bzw. Dispergierhilfsmittel.

Als nicht-ionische Tenside bzw. Dispergierhilfsmittel kommen alle üblicherweise in agrochemischen Mitteln einsetzbaren Stoffe dieses Typs in Betracht. Vorzugsweise genannt seien Polyethylenoxid-polypropylenoxid-Blockcopolymere, Polyethylenglykolether von linearen Alkoholen, Umsetzungsprodukte von Fettsäuren mit Ethylenoxid und/oder Propylenoxid, ferner Polyvinylalkohol, Polyvinylpyrrolidon, Mischpolymerisate aus Polyvinylalkohol und Polyvinylpyrrolidon sowie Copolymerisate aus (Meth)acrylsäure und (Meth)acrylsäureestern, weiterhin Alkylethoxylate und Alkylarylethoxylate, die gegebenenfalls phosphatiert und gegebenenfalls mit Basen neutralisiert sein können, wobei Sorbitolethoxylate beispielhaft genannt seien, sowie Polyoxyalkylenamin-Derivate.

Als anionische Tenside kommen alle üblicherweise in agrochemischen Mitteln einsetzbaren Substanzen dieses Typs in Frage. Bevorzugt sind Alkalimetall- und Erdalkalimetall-Salze von Alkylsulfonsäuren oder Alkylarylsulfonsäuren.

Eine weitere bevorzugte Gruppe von anionischen Tensiden bzw. Dispergierhilfsmitteln sind in Pflanzenöl wenig lösliche Salze von Polystyrolsulfonsäuren, Salze von Polyvinylsulfonsäuren, Salze von Naphthalinsulfonsäure-Formaldehyd-Kondensationsprodukten, Salze von Kondensationsprodukten aus Naphthalinsulfonsäure, Phenolsulfonsäure und Formaldehyd sowie Salze von Ligninsulfonsäure.

Als Zusatzstoffe, die in den erfindungsgemäßen Formulierungen enthalten sein können, kommen Emulgatoren, schaumhemmende Mittel, Konservierungsmittel, Antioxydantien, Spreitmittel, Farbstoffe und Verdickungsmittel in Betracht.

Bevorzugte Emulgatoren sind ethoxylierte Nonylphenole, Umsetzungsprodukte von Alkylphenolen mit Ethylenoxid und/oder Propylenoxid, ethoxylierte Arylalkylphenole, weiterhin ethoxylierte und propoxylierte Arylalkylphenole, sowie sulfatierte oder phosphatierte Arylalkylethoxylate bzw. -ethoxy-propoxylate, wobei Sorbitan-Derivate, wie Polyethylenoxid-Sorbitan-Fettsäureester und Sorbitan-Fettsäureester, beispielhaft genannt seien.

Als schaumhemmende Stoffe kommen alle üblicherweise für diesen Zweck in agrochemischen Mitteln einsetzbaren Substanzen in Betracht. Bevorzugt sind Silikonöle und Magnesiumstearat.

Als Konservierungsmittel kommen alle üblicherweise für diesen Zweck in agrochemischen Mitteln dieses Typs einsetzbaren Substanzen in Frage. Als Beispiele genannt seien Preventol® (Fa. Bayer AG) und Proxel®.

Als Antioxydantien kommen alle üblicherweise für diesen Zweck in agrochemischen Mitteln einsetzbaren Substanzen in Betracht. Bevorzugt sind Butylhydroxytoluol und/oder Zitronensäure.

Als Spreitmittel kommen alle üblicherweise für diesen Zweck in agro-chemischen Mitteln einsetzbaren Substanzen in Betracht. Bevorzugt sind Alkylsiloxane.

Als Farbstoffe kommen alle üblicherweise für diesen Zweck in agrochemischen Mitteln einsetzbaren Substanzen in Frage. Beispielhaft genannt seien Titandioxid, Farbruß, Zinkoxid und Blaupigmente sowie Permanentrot FGR.

Als Verdickungsmittel kommen alle üblicherweise für diesen Zweck in agrochemischen Mitteln einsetzbaren Substanzen in Betracht, die als Verdickungsmittel fungieren. Bevorzugt sind anorganische Partikel, wie Carbonate, Silikate und Oxide, sowie auch organische Substanzen, wie Harnstoff-Formaldehyd-Kondensate. Beispielhaft erwähnt seien Kaolin, Rutil, Siliciumdioxid, sogenannte hochdisperse Kieselsäure, Kieselgele, sowie natürliche und synthetische Silikate, außerdem Talkum.

Der erfindungsgemäßen Formulierungen können in einer besondereren Ausführungsform noch mindestens einen weiteren Wirkstoff (Insektizide, Lockstoffe, Sterilantien, Bakterizide, Akarizide, Nematizide, Fungizide, wachstumsregulierenden Stoffe oder Herbizide) enthalten. Zu den Insektiziden zählen beispielsweise Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:

### Fungizide:

Inhibitoren der Nucleinsäure Synthese
   Benalaxyl, Benalaxyl-M, Bupirimat, Chiralaxyl, Clozylacon, Dimethirimol, Ethirimol, Furalaxyl, Hymexazol, Metalaxyl, Metalaxyl-M, Ofurace, Oxadixyl, Oxolinsäure
Inhibitoren der Mitose und Zellteilung
   Benomyl, Carbendazim, Diethofencarb, Fuberidazole, Pencycuron, Thiabendazol, Thiophanat-methyl, Zoxamid
Inhibitoren der Atmungskette Komplex I
   Diflumetorim
Inhibitoren der Atmungskette Komplex II
   Boscalid, Carboxin, Fenfuram, Flutolanil, Furametpyr, Mepronil, Oxycarboxin, Penthiopyrad, Thifluzamid
Inhibitoren der Atmungskette Komplex III
   Azoxystrobin, Cyazofamid, Dimoxystrobin, Enestrobin, Famoxadon, Fenamidon, Fluoxastrobin, Kresoximmethyl, Metominostrobin, Orysastrobin, Pyraclostrobin, Picoxystrobin
Entkoppler
   Dinocap, Fluazinam
Inhibitoren der ATP Produktion
   Fentinacetat, Fentinchlorid, Fentinhydroxid, Silthiofam
Inhibitoren der Aminosäure- und Proteinbiosynthese
   Andoprim, Blasticidin-S, Cyprodinil, Kasugamycin, Kasugamycinhydrochlorid Hydrat, Mepanipyrim, Pyrimethanil
Inhibitoren der Signal-Transduktion
   Fenpiclonil, Fludioxonil, Quinoxyfen
Inhibitoren der Fett- und Membran Synthese
   Chlozolinat, Iprodion, Procymidon, Vinclozolin
   Ampropylfos, Kalium-Ampropylfos, Edifenphos, Iprobenfos (IBP), Isoprothiolan, Pyrazophos
   Tolclofos-methyl, Biphenyl
   Iodocarb, Propamocarb, Propamocarb hydrochlorid
Inhibitoren der Ergosterol Biosynthese
   Fenhexamid,
   Azaconazol, Bitertanol, Bromuconazol, Cyproconazol, Diclobutrazol, Difenoconazol, Diniconazol, Diniconazol-M, Epoxiconazol, Etaconazol, Fenbuconazol, Fluquinconazol, Flusilazol, Flutriafol, Furconazol, Furconazol-cis, Hexaconazol, Imibenconazol, Ipconazol, Metconazol, Myclobutanil, Paclobutrazol, Penconazol, Propiconazol, Prothioconazol, Simeconazol, Tebuconazol, Tetraconazol, Triadimefon, Triadimenol, Triticonazol, Uniconazol, Voriconazol, Imazalil, Imazalilsulfat, Oxpoconazol, Fenarimol, Flurprimidol, Nuarimol, Pyrifenox, Triforin, Pefurazoat, Prochloraz, Triflumizol, Viniconazol,
   Aldimorph, Dodemorph, Dodemorphacetat, Fenpropimorph, Tridemorph, Fenpropidin, Spiroxamin,
   Naftifin, Pyributicarb, Terbinafin
Inhibitoren der Zellwand Synthese
   Benthiavalicarb, Bialaphos, Dimethomorph, Flumorph, Iprovalicarb, Polyoxins, Polyoxorim, Validamycin A
Inhibitoren der Melanin Biosynthese
   Capropamid, Diclocymet, Fenoxanil, Phtalid, Pyroquilon, Tricyclazol
Resistenzinduktion
   Acibenzolar-S-methyl, Probenazol, Tiadinil
Multisite
   Captafol, Captan, Chlorothalonil, Kupfersalze wie: Kupferhydroxid, Kupfernaphthenat, Küpferoxychlorid, Kupfersulfat, Kupferoxid, Oxin-Kupfer und Bordeaux Mischung, Dichlofluanid, Dithianon, Dodin, Dodin freie Base, Ferbam, Fluorofolpet, Guazatin, Guazatinacetat, Iminoctadin, Iminoctadinalbesilat, Iminoctadintriacetat, Mankupfer, Mancozeb, Maneb, Metiram, Metiram Zink, Propineb, Schwefel und Schwefelpräparate enthaltend Calciumpolysulphid, Thiram, Tolylfluanid, Zineb, Ziram
Unbekannter Mechanismus
   Amibromdol, Benthiazol, Bethoxazin, Capsimycin, Carvon, Chinomethionat, Chloropicrin, Cufraneb, Cyflufenamid, Cymoxanil, Dazomet, Debacarb, Diclomezine, Dichlorophen, Dicloran, Difenzoquat, Difenzoquat Methylsulphat, Diphenylamin, Ethaboxam, Ferimzon, flumetover, Flusulfamid, Fluopicolid, Fluoroimid, Hexachlorobenzol, 8-Hydroxychinolinsulfat, Irumamycin, Methasulphocarb, Metrafenon, Methyl Isothiocyanat, Mildiomycin, Natamycin, Nickel dimethyldithiocarbamat, Nitrothal-isopropyl, Octhilinon, Oxamocarb, Oxyfenthiin, Pentachlorophenol und Salze, 2-Phenylphenol und Salze, Piperalin, Propanosin -Natrium, Proquinazid, Pyrrolnitrin, Quintozen, Tecloftalam, Tecnazen, Triazoxid, Trichlamid, Zarilamid und 2,3,5,6-Tetrachlor-4-(methylsulfonyl)-pyridin, N-(4-Chlor-2-nitrophenyl)-N-ethyl-4-methyl-benzenesulfonamid, 2-Amino-4-methyl-N-phenyl-5-thiazole-carboxamid, 2-Chlor-N-(2,3-dihydro-1,1,3-trimethyl-1H-inden-4-yl)-3-pyridincarboxamid, 3-[5-(4-Chlorphenyl)-2,3-dimethylisoxazolidin-3-yl]pyridin, cis-1-(4-Chlorphenyl)-2-(1H-1,2,4-triazol-1-yl)-cycloheptanol, 2,4-Dihydro-5-methoxy-2-methyl-4-[[[[1-[3-(trifluoromethyl)-phenyl]-ethyliden]-amino]-oxy]-methyl]-phenyl]-3H-1,2,3-triazol-3-on (185336-79-2), Methyl 1-(2,3-dihydro-2,2-dimethyl-1H-inden-1-yl)-1H-imidazole-5-carboxylat, 3,4,5-Trichlor-2,6-pyridindicarbonitril, Methyl 2-[[[cyclopropyl[(4-methoxyphenyl) imino]methyl]thio]methyl]-.alpha.-(methoxymethylen)- benzacetat, 4-Chlor-alpha-propinyloxy-N-[2-[3-methoxy-4-(2-propinyloxy)phenyl]ethyl]-benzacetamide, (2S)-N-[2-[4-[[3-(4-chlorophenyl)-2-propinyl]oxy]-3-methoxyphenyl]ethyl]-3-methyl-2-[(methylsulfonyl)amino]-butan-amid, 5-Chlor-7-(4-methylpiperidin-1-yl)-6-(2,4,6-trifluorophenyl)[1,2,4]triazolo[1,5-a]pyrimidin, 5-Chlor-6-(2,4,6-trifluorophenyl)-N-[(1R)-1,2,2-trimethylpropyl][1,2,4]triazolo[1,5-a]pyrimidin-7-amin, 5-Chlor-N-[(1R)-1,2-dimethylpropyl]-6-(2,4,6-trifluorophenyl) [1,2,4]-triazolo[1,5-a]pyrimidin-7-amine, N-[1-(5-Brom-3-chloropyridin-2-yl)ethyl]-2,4-dichloro-nicotinamid, N-(5-Brom-3-chlorpyridin-2-yl)methyl-2,4-dichlornicotinamid, 2-Butoxy-6-iod-3-propyl-benzopyranon-4-on, N-{(Z)-[(cyclopropylmethoxy) imino][6-(difluormethoxy)-2,3-difluorphenyl]methyl}-2-benzacetamid, N-(3-Ethyl-3,5,5-trimethyl-cyclohexyl)-3-formylamino-2-hydroxy-benzamid, 2-[[[[1-[3(1Fluor-2-phenylethyl)oxy]phenyl] ethyliden]amino]oxy]methyl]-alpha-(methoxyimino)-N-methyl-alphaE-benzacetamid, N-{2-[3-Chlor-5-(trifluormethyl)pyridin-2-yl]ethyl}-2-(trifluoromethyl)benzamid, N-(3',4'-dichlor-5-fluorbiphenyl-2-yl)-3-(difluormethyl)-1-methyl-1H-pyrazol-4-carboxamid, N-(6-Methoxy-3-pyridinyl)-cyclopropan carboxamid, 1-[(4-Methoxyphenoxy)methyl]-2,2-dimethylpropyl-1H-imidazol-1- carbonsäure, O-[1-[(4-Methoxyphenoxy)methyl]-2,2-dimethylpropyl]-1H-imidazol- 1- carbothioic acid, 2-(2-{[6-(3-Chlor-2-methylphenoxy)-5-fluorpyrimidin-4-yl]oxy}-phenyl)-2-(methoxyimino)-N-methylacetamid

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Acetylcholinesterase (AChE) Inhibitoren
   Carbamate,
      zum Beispiel Alanycarb, Aldicarb, Aldoxycarb, Allyxycarb, Aminocarb, Bendiocarb, Benfuracarb, Bufencarb, Butacarb, Butocarboxim, Butoxycarboxim, Carbaryl, Carbofuran, Carbosulfan, Cloethocarb, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Trimethacarb, XMC, Xylylcarb, Triazamate
   Organophosphate,
      zum Beispiel Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos(-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/ -ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion
Natrium-Kanal-Modulatoren / Spannungsabhängige Natrium-Kanal-Blocker
   DDT

   Oxadiazine,
   zum Beispiel Indoxacarb
Acetylcholin-Rezeptor-Agonisten/-Antagonisten
   Chloronikotinyle,
      zum Beispiel Acetamiprid, Clothianidin, Dinotefuran, Imidacloprid, Nitenpyram, Nithiazine, Thiacloprid, Thiamethoxam
   Nicotine, Bensultap, Cartap
Acetylcholin-Rezeptor-Modulatoren
   Spinosyne,
   zum Beispiel Spinosad
GABA-gesteuerte Chlorid-Kanal-Antagonisten
   Organochlorine,
      zum Beispiel Camphechlor, Chlordane, Endosulfan, Gamma-HCH, HCH, Heptachlor, Lindane, Methoxychlor
   Fiprole,
      zum Beispiel Acetoprole, Ethiprole, Fipronil, Pyrafluprole, Pyriprole, Vaniliprole
Chlorid-Kanal-Aktivatoren
   Mectine,
      zum Beispiel Avermectin, Emamectin, Emamectin-benzoate, Ivermectin, Milbemycin
Juvenilhormon-Mimetika,
   zum Beispiel Diofenolan, Epofenonane, Fenoxycarb, Hydroprene, Kinoprene, Methoprene, Pyriproxifen, Triprene
Ecdysonagonisten/disruptoren
   Diacylhydrazine,
      zum Beispiel Chromafenozide, Halofenozide, Methoxyfenozide, Tebufenozide
Inhibitoren der Chitinbiosynthese
   Benzoylharnstoffe,
      zum Beispiel Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron
   Buprofezin
   Cyromazine
Inhibitoren der oxidativen Phosphorylierung, ATP-Disruptoren
   Diafenthiuron
   Organozinnverbindungen,
      zum Beispiel Azocyclotin, Cyhexatin, Fenbutatin-oxide
Entkoppler der oxidativen Phoshorylierung durch Unterbrechung des H-Protongradienten
   Pyrrole,
      zum Beispiel Chlorfenapyr
   Dinitrophenole,
      zum Beispiel Binapacyrl, Dinobuton, Dinocap, DNOC
Seite-I-Elektronentransportinhibitoren
   METI's,
      zum Beispiel Fenazaquin, Fenpyroximate, Pyrimidifen, Pyridaben, Tebufenpyrad, Tolfenpyrad
   Hydramethylnon
   Dicofol
Seite-II-Elektronentransportinhibitoren
   Rotenone
Seite-III-Elektronentransportinhibitoren
   Acequinocyl, Fluacrypyrim
Mikrobielle Disruptoren der Insektendarmmembran
   Bacillus thuringiensis-Stämme
Inhibitoren der Fettsynthese
   Tetronsäuren,
   zum Beispiel Spirodiclofen, Spiromesifen
   Tetramsäuren,
   zum Beispiel Spirotetramat (CAS-Reg.-No.: 203313-25-1) und 3-(2,5-Dimethylphenyl)-8-methoxy-2-oxo-1-azaspiro[4.5]dec-3-en-4-yl ethyl carbonäte (alias: Carbonic acid, 3-(2,5-dimethylphenyl)-8-methoxy-2-oxo-1-azaspiro[4.5]dec-3-en-4-yl ethyl ester, CAS-Reg.-No.: 382608-10-8)
   Carboxamide,
   zum Beispiel Flonicamid
   Oktopaminerge Agonisten,
   zum Beispiel Amitraz
Inhibitoren der Magnesium-stimulierten ATPase,
   Propargite
   Benzoesäuredicarboxamide,
   zum Beispiel Flubendiamide
   Nereistoxin-Analoge,
   zum Beispiel Thiocyclam hydrogen oxalate, Thiosultap-sodium
Biologika, Hormone oder Pheromone
   Azadirachtin, Bacillus spec., Beauveria spec., Codlemone, Metarrhizium spec., Paecilomyces spec., Thuringiensin, Verticillium spec.
Wirkstoffe mit unbekannten oder nicht spezifischen Wirkmechanismen
   Begasungsmittel,
      zum Beispiel Aluminium phosphide, Methyl bromide, Sulfuryl fluoride
   Fraßhemmer,
      zum Beispiel Cryolite, Flonicamid, Pymetrozine
   Milbenwachstumsinhibitoren,
      zum Beispiel Clofentezine, Etoxazole, Hexythiazox
   Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyridalyl, Sulfluramid, Tetradifon, Tetrasul, Tri-arathene,Verbutin

Der Gehalt an den einzelnen Komponenten kann in den erfindungsgemäßen Suspensionskonzentraten auf Ölbasis innerhalb eines größeren Bereiches variiert werden. So liegen die Konzentrationen
- an agrochemischen Wirkstoffen zwischen 5 und 40 Gew.-%, vorzugsweise zwischen 10 und 37,5 Gew.-%, ganz besonders bevorzugt zwischen 12,5 und 35 Gew.-%,
- an Penetrationsförderer zwischen 5 und 55 Gew.-%, vorzugsweise zwischen 10 und 35 Gew.-%,
- an Pflanzenöl zwischen 15 und 55 Gew.-%, vorzugsweise zwischen 20 und 50 Gew.-%,
- Cyclohexanon zwischen 5 und 20 Gew.-%, vorzugsweise zwischen 7 und 16 Gew.-%,
- an Tensiden bzw. Dispergierhilfsmitteln zwischen 2,5 und 30 Gew.-%, vorzugsweise zwischen 5,0 und 25 Gew.-% und
- an Zusatzstoffen zwischen 0,1 und 25 Gew.-%, vorzugsweise zwischen 0,1 und 20 Gew.-%.

Die Herstellung der erfindungsgemäßen Suspensionskonzentrate auf Ölbasis erfolgt in der Weise, dass man die Komponenten in den jeweils gewünschten Verhältnissen miteinander vermischt. Die Reihenfolge, in der die Bestandteile miteinander vermengt werden, ist beliebig. Zweckmäßigerweise setzt man die festen Komponenten in feingemahlenem Zustand ein. Es ist aber auch möglich, die nach dem Vermengen der Bestandteile entstehende Suspension zunächst einer Grob- und dann einer Feinmahlung zu unterziehen, so dass die mittlere Teilchengröße unterhalb von 20 µm liegt. Bevorzugt sind Suspensionskonzentrate, in denen die festen Partikel eine mittlere Teilchengröße zwischen 1 und 10 µm aufweisen.

Die Temperaturen können bei der Durchführung des erfindungsgemäßen Verfahrens in einem bestimmten Bereich variiert werden. Man arbeitet im allgemeinen bei Temperaturen zwischen 10°C und 60°C, vorzugsweise zwischen 15°C und 40°C.

Zur Durchführung des erfindungsgemäßen Verfahrens kommen übliche Misch- und Mahlgeräte in Betracht, die zur Herstellung von agrochemischen Formulierungen eingesetzt werden.

Bei den erfindungsgemäßen Suspensionskonzentraten auf Ölbasis handelt es sich um Formulierungen, die auch nach längerer Lagerung bei erhöhten Temperaturen oder in der Kälte stabil bleiben, da kein signifikantes Kristallwachstum beobachtet wird. Sie lassen sich durch Verdünnen mit Wasser in homogene Spritzflüssigkeiten überführen. Die Anwendung dieser Spritzflüssigkeiten erfolgt nach üblichen Methoden, also zum Beispiel durch Verspritzen, Gießen oder Injizieren.

Die Aufwandmenge an den erfindungsgemäßen Suspensionskonzentraten auf Ölbasis kann innerhalb eines größeren Bereiches variiert werden. Sie richtet sich nach den jeweiligen agrochemischen Wirkstoffen und nach deren Gehalt in den Formulierungen.

Mit Hilfe der erfindungsgemäßen Suspensionskonzentrate auf Ölbasis lassen sich agrochemische Wirkstoffe, insbesondere aus der Reihe der Neonikotinoide, in besonders vorteilhafter Weise auf Pflanzen und/oder deren Lebensraum ausbringen.

Mit den erfindungsgemäßen Formulierungen können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome ausgerührt werden. Zu den Pflanzenteilen gehört auch Erntegut sowie vegetatives und generatives Vermehrungsmaterial, beispielsweise Stecklinge, Knollen, Rhizome, Ableger und Samen.

Hervorgehoben sei hierbei die besonders vorteilhafte Wirkung der erfindungsgemäßen Mittel hinsichtlich der Anwendung in Getreidepflanzen, wie z.B. Weizen, Hafer, Gerste, Dinkel, Triticale und Roggen, aber auch in Mais, Hirse, Reis, Zuckerrohr, Soja, Sonnenblumen, Kartoffeln, Baumwolle, Raps, Canola, Tabak, Zuckerrüben Futterrüben, Spargel, Hopfen sowie Obstpflanzen (umfassend Kernobst wie z.B. Äpfel und Birnen, Steinobst wie z.B. Pfirsiche, Nektarinen, Kirschen, Pflaumen und Aprikosen, Zitrusfrüchte wie z.B. Orangen, Grapefruits, Limetten, Zitronen, Kumquats, Mandarinen und Satsumas, Nüsse wie z.B. Pistazien, Mandeln, Walnüsse und Pecannüsse, tropische Früchte wie z.B. Mango, Papaya, Ananas, Datteln und Bananen, und Weintrauben) und Gemüse (umfassend Blattgemüse, wie z.B. Endivien, Feldsalat, Knollenfenchel, Kopf- und Pflücksalate, Mangold, Spinat und Zichoriensalat, Kohlgemüse wie z.B. Blumenkohl, Brokkoli, Chinakohl, Grünkohl (Winter- oder Krauskohl), Kohlrabi, Rosenkohl, Rotkohl, Weißkohl und Wirsing, Fruchtgemüse wie z.B. Auberginen, Gurken, Paprika, Speisekürbisse, Tomaten, Zucchini und Zuckermais, Wurzelgemüse wie z.B. Knollensellerie, Mairüben, Möhren, Gelbe Rüben, Radieschen, Rettich, Rote Rüben, Schwarzwurzeln und Stangensellerie, Hülsenfrüchte wie z.B. Erbsen und Bohnen sowie Zwiebelgemüse wie z.B. Lauch und Speisezwiebeln).

Die erfindungsgemäße Behandlung der Pflanzen und Pflanzenteile mit den erfindungsgemäßen Formulierungen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die enthaltenen agrochemischen Wirkstoffe entfalten dabei eine bessere biologische Wirksamkeit als bei Applikation in Form der entsprechenden herkömmlichen Formulierungen.

Die Erfindung wird durch die folgenden Beispiele veranschaulicht.

### Beispiele

### Herstellungsbeispiele

### Beispiel 1

Zur Herstellung eines Suspensionskonzentrates werden

| | |
|---|---|
| 144,0 | g Imidacloprid |
| 38,4 | g Deltamethrin |
| 100,0 | g Arlatone ® T |
| 75,0 | g Cyclohexanon |
| 130,0 | g Atlox ® 3467 |
| 20,0 | g Ligninsulfonat (Borresperse ® NA) |
| 25,0 | g Propylenglycol |
| 0,5 | g Polydimethylsiloxan |
| 2,0 | g wasserfreie Zitronensäure |
| 2,0 | g 2-6-Di-tert-butyl-4-methylphenol |

unter Rühren bei Raumtemperatur in ein Gemisch aus

| | |
|---|---|
| 200,0 | g der Verbindung der Formel (I-c-1) und |
| 263,1 | g Sonnenblumenöl |

gegeben. Nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90 % der Feststoffpartikel eine Teilchengröße unter 6 µm aufweisen.

Analog zu Beispiel 1 sind folgende Rezepturen hergestellt worden

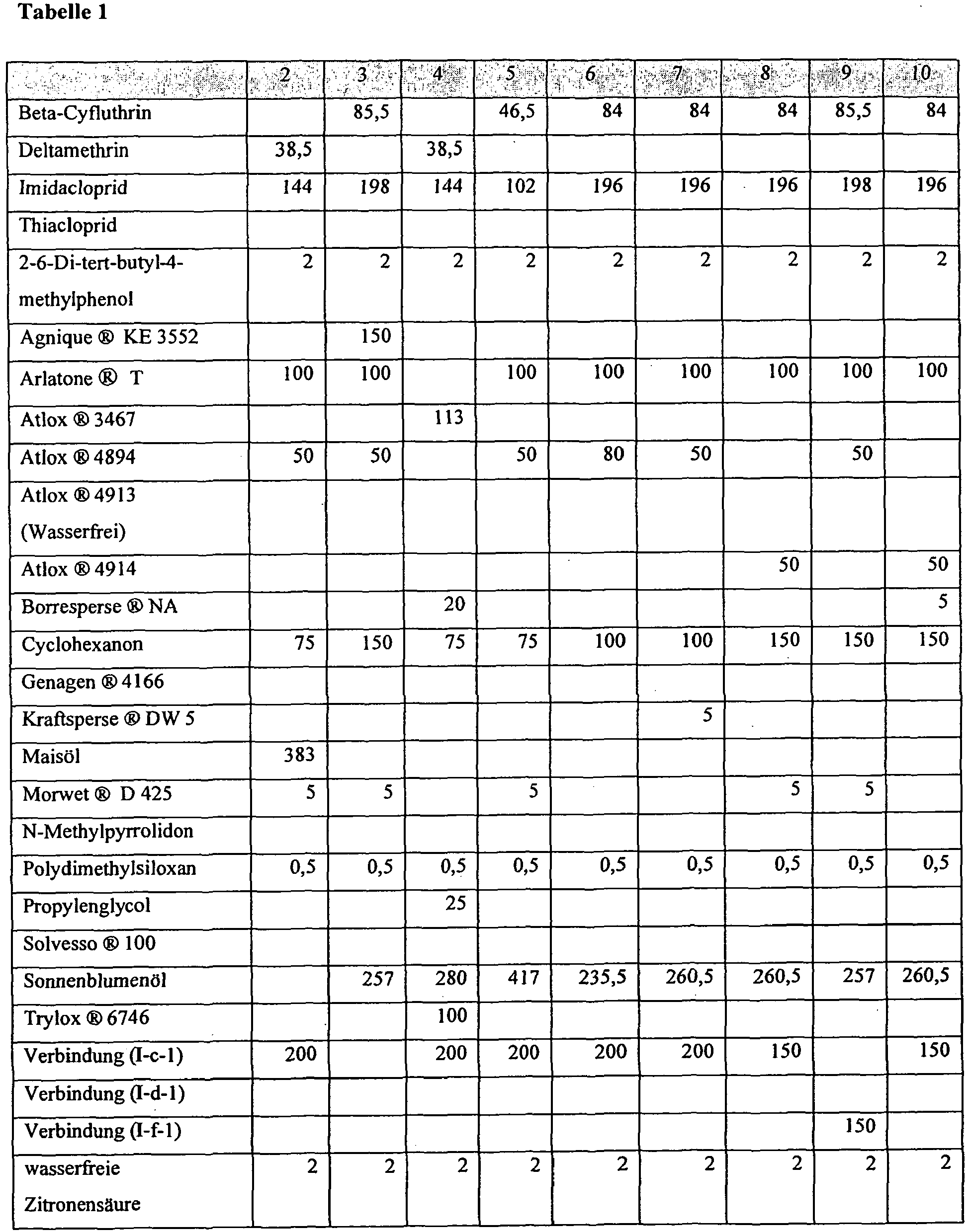

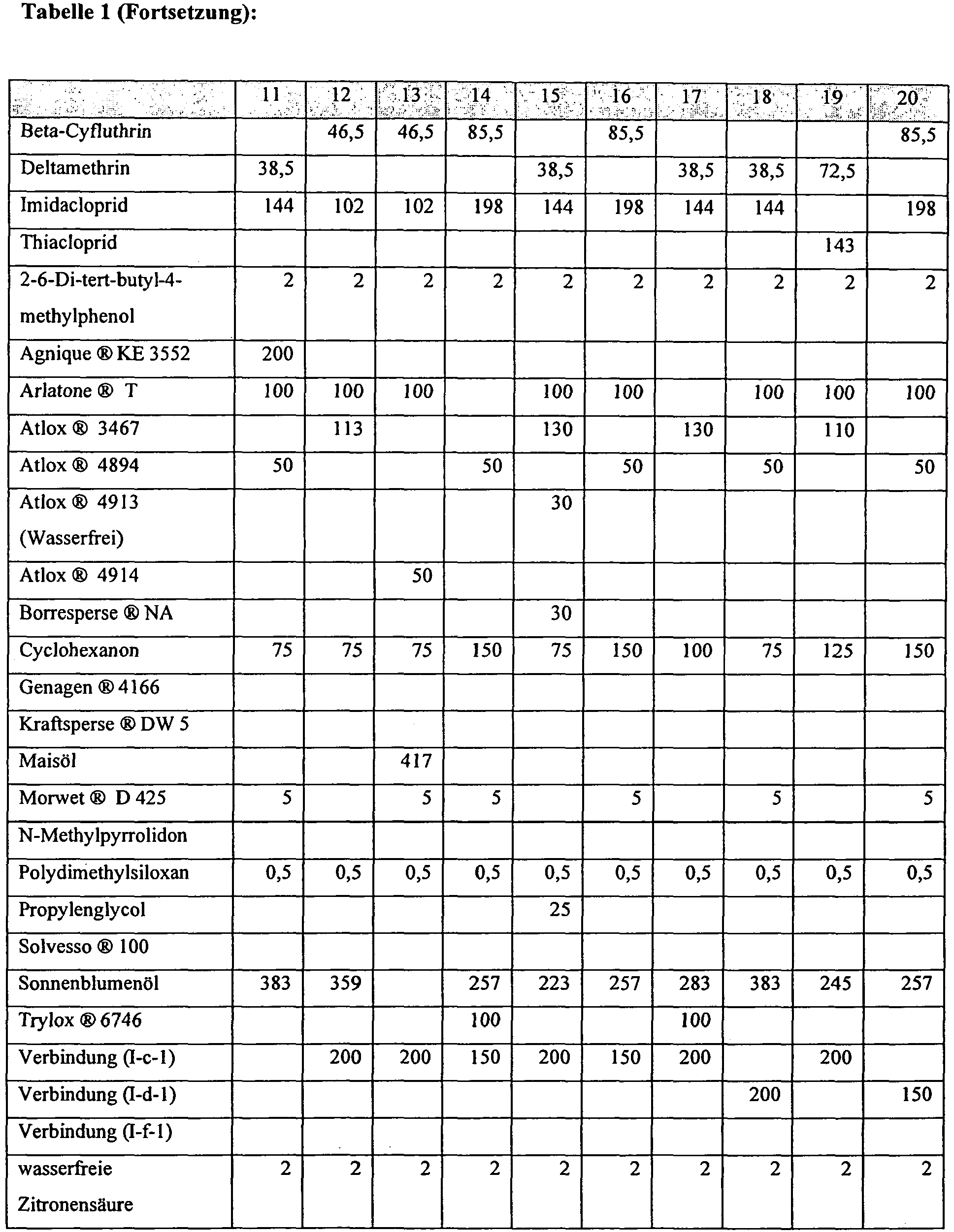

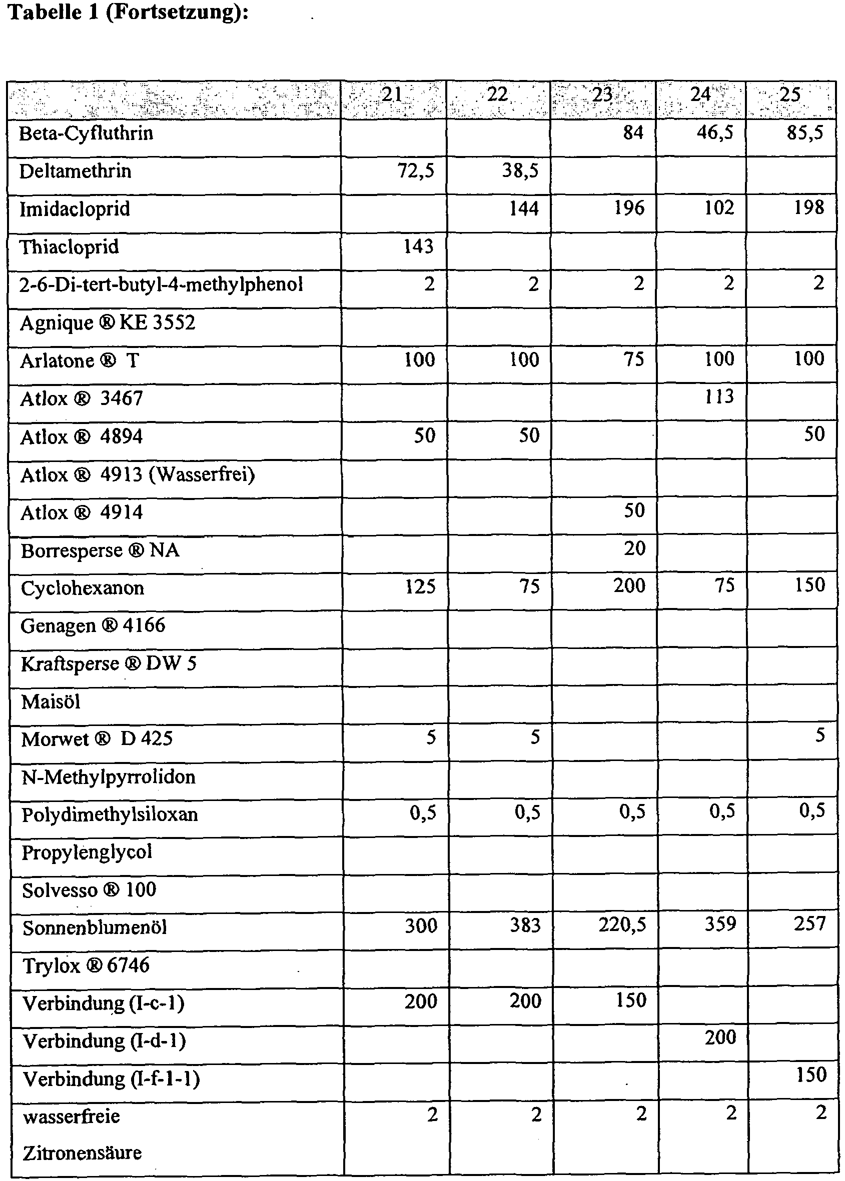

### Vergleichsbeispiele

Analog zu Beispiel 1 sind folgende Rezepturen hergestellt worden

**Tabelle 2**

| | 1 | 2 | 3 | 4 | 5 | 6 | 7 |
|---|---|---|---|---|---|---|---|
| Beta-Cyfluthrin | | | 94,5 | 46,5 | 85,5 | 85,5 | 85,5 |
| Deltamethrin | 39,5 | 94,5 | | | | | |
| Imidacloprid | 147 | 187 | 187 | 102 | 198 | 198 | 198 |
| Thiacloprid | | | | | | | |
| 2-6-Di-tert-butyl-4-methylphenol | 2 | 2 | 2 | 2 | 2 | 2 | 2 |
| Agnique ® KE 3552 | | | | | | | |
| Arlatone ® T | 100 | 100 | 100 | 100 | 100 | 100 | 100 |
| Atlox ® 3467 | 130 | 130 | 130 | 113 | | | |
| Atlox ® 4894 | | | | | 50 | 50 | 50 |
| Atlox ® 4913 (Wasserfrei) | | | | | | | |
| Atlox ® 4914 | | | | | | | |
| Borresperse ® NA | | 20 | | | | | |
| Cyclohexanon | | | | | | | |
| Genagen ® 4166 | | | | | | | 150 |
| Kraftsperse ® DW 5 | | | | | | | |
| Maisöl | | | | | | | |
| Morwet ® D 425 | | | 20 | | 5 | 5 | 5 |
| N-Methylpyrrolidon | | | | | | 150 | |
| Polydimethylsiloxan | 0,5 | 0,5 | 0,5 | 0,5 | 0,5 | 0,5 | 0,5 |
| Propylenglycol | | | | | | | |
| Solvesso ® 100 | | | | 75 | 150 | | |
| Sonnenblumenöl | 379 | 264 | 264 | 359 | 257 | 257 | 257 |
| Trylox ® 6746 | | | | | | | |
| Verbindung (I-c-1) | 200 | 200 | 200 | 200 | 150 | 150 | 150 |
| Verbindung (I-d-1) | | | | | | | |
| Verbindung (I-f-1) | | | | | | | |
| wasserfreie Zitronensäure | 2 | 2 | 2 | 2 | 2 | 2 | 2 |

Die durch ihre Handelsnamen definierten Komponenten der erfindungsgemäßen Zusammensetzungen sind bei folgenden Lieferanten erhältlich:

| Handelsname | Verbindungstyp | Lieferant |
|---|---|---|
| Agnique ® KE 3552 | Alkanol-Alkoxylat | Cognis |
| Arlatone ® T | PEG-40 sorbitan peroleate, nicht-ionisch | Uniqema |
| Atlox ® 3467 | Blend containing Alkylaryl sulphonate, Ethylhexanol, Ethoxylated Alcohol | Uniqema |
| Atlox ® 4894 | Polyalkoxylated alcohol | Uniqema |
| Atlox ® 4913 (Wasserfrei) | Polymeres nichtionisches Tensid | Uniqema |
| Atlox ® 4914 | Polymeric nonionic surfactant | Uniqema |
| Borresperse ® NA | Ligninsulfonat | Borregaard LignoTech |
| Genagen ® 4166 | Caprylic/capric fatty acid dimethylamide | Clariant |
| Kraftsperse ® DW 5 | Ligninsulfonat, Natriumsalz | Westvaco |
| Morwet ® D 425 | Naphthalinsulfonat | Witco |
| Solvesso ® 100 | Aromatisches organisches Lösungsmittel | Exxon Mobile |
| Trylox ® 6746 | PEG-40 sorbitol hexaoleate | Cognis |

### Kristallisationsverhalten

Zur Untersuchung des Kristallisationsverhaltens werden 100 ml Formulierung acht Wochen unter wechselnde Temperaturbedingungen gelagert. Die Temperaturbedingungen sind
- 48 Stunden bei 30 °C,
- in 22, 5 Stunden Verringern der Temperatur mit 2 °C / Stunde bis- 15 °C,
- 75 Stunden bei - 15 °C,
- in 22, 5 Stunden Erhöhen der Temperatur mit 2 °C / Stunde bis 30 °C.

Anschließend an die Lagerung wird die Probe auf Raumtemperatur gebracht und das Kristallisationsverhalten wird überprüft.

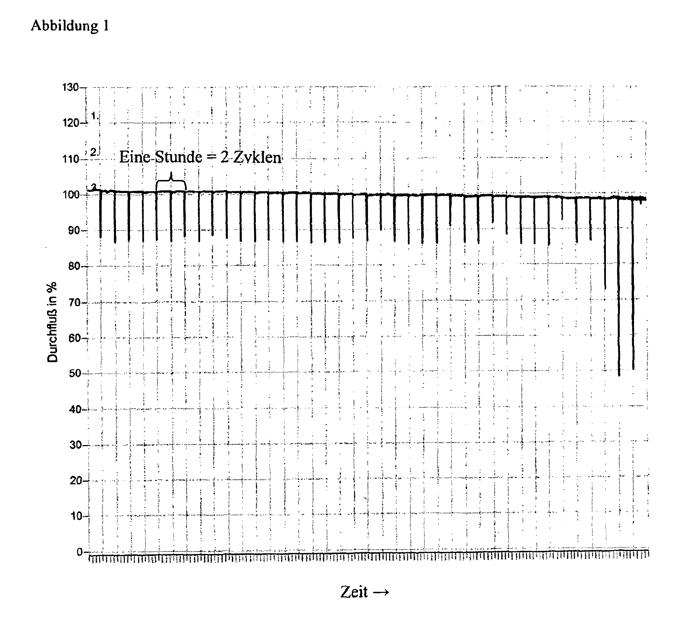

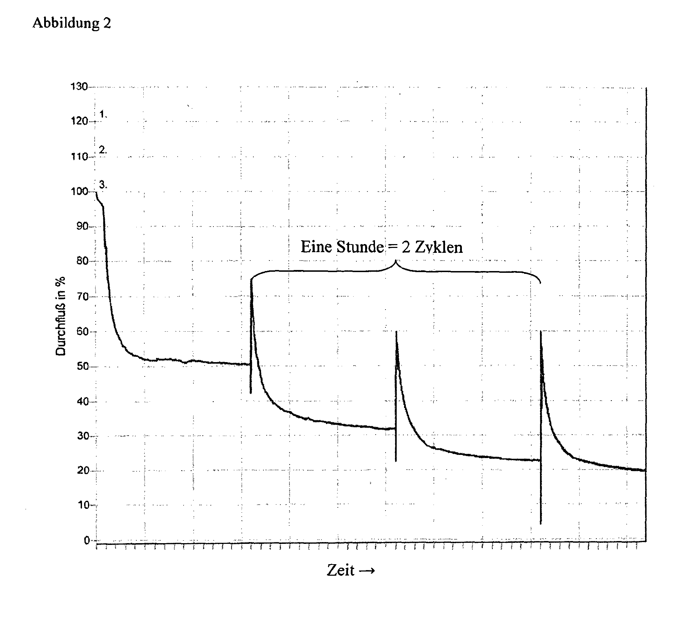

Zur Prüfung der Kristallisationseigenschaften werden jeweils 500 ml einer wässerigen Spritzbrühe, die einen Konzentratgehalt von 0,5 Gew.-% aufweisen, in einer Durchflussapparatur mit Hilfe einer Pumpe 30 Minuten lang durch ein feinmaschiges Sieb umgepumpt. Während dieser Prozedur wird der Durchfluss über das Sieb gemessen. Bei gleich bleibendem Durchfluss werden vierzig Wiederholungen dieser Vorgang mit jeweils 500 ml frisch eingesetzter Spritzbrühe durchgeführt. Kristallwachstum in den geprüften Formulierungen wird zum Blockieren des Siebs führen and dadurch einer Durchflussverlust über das Sieb verursachen. Bei einem Durchfluss unter 20% wird der Messungszyklus abgebrochen. Beispielhaft sind 2 Ergebnisse als Grafik wiedergegeben. Grafik 1 zeigt das Ergebnis einen Durchflusstest mit einer erfindungsgemäßen Formulierung, bei der nach vierzig Zyklen (20 Stunden) der Durchfluss noch unverändert ist. Grafik 2 zeigt das Ergebnis für eine Vergleichsrezeptur. Nach vier Zyklen (2 Stunden) ist der Durchfluss auf 20 % gefallen (siehe Abbildungen 1 und 2).
- Abbildung 1:: Ergebnis eines Durchflusstests mit der erfindungsgemäßen Formulierung 16, gemessen über 40 Zyklen
- Abbildung 2:: Ergebnis eines Durchflusstests mit der Vergleichsformulierung 3, gemessen über 4 Zyklen

### Verwendungsbeispiel II: Kristallisationsverhalten

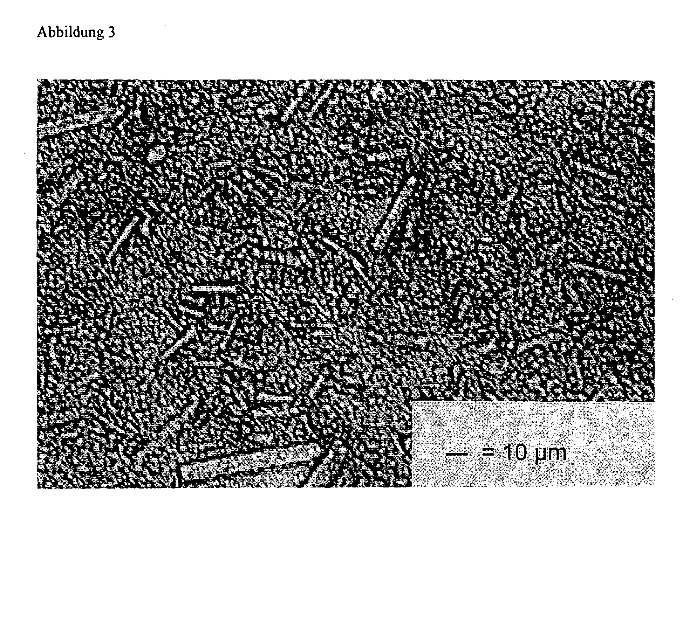

Nach acht Wochen Lagerung der Formulierung unter wechselnden Temperaturbedingungen und bei 54 °C wird das Wachsen der Wirkstoffkristalle mittels Lichtmikroskopie bestimmt. Direkt nach Herstellung zeigen alle Formulierungen Partikelgrößen bis 10 Mikrometer. Alle erfindungsgemäßen Formulierungen zeigen nach Lagerung Partikelgrößen bis maximal 20 Mikrometer. Die Vergleichsformulierungen zeigen erheblich gröbere Teilchen, bis über 100 Mikrometer (siehe Abbildungen 3 bis 5).
- Abbildung 3:: Lichtmikroskopische Untersuchung von Vergleichsbeispiel 3 nach oben beschriebener achtwöchiger Lagerung

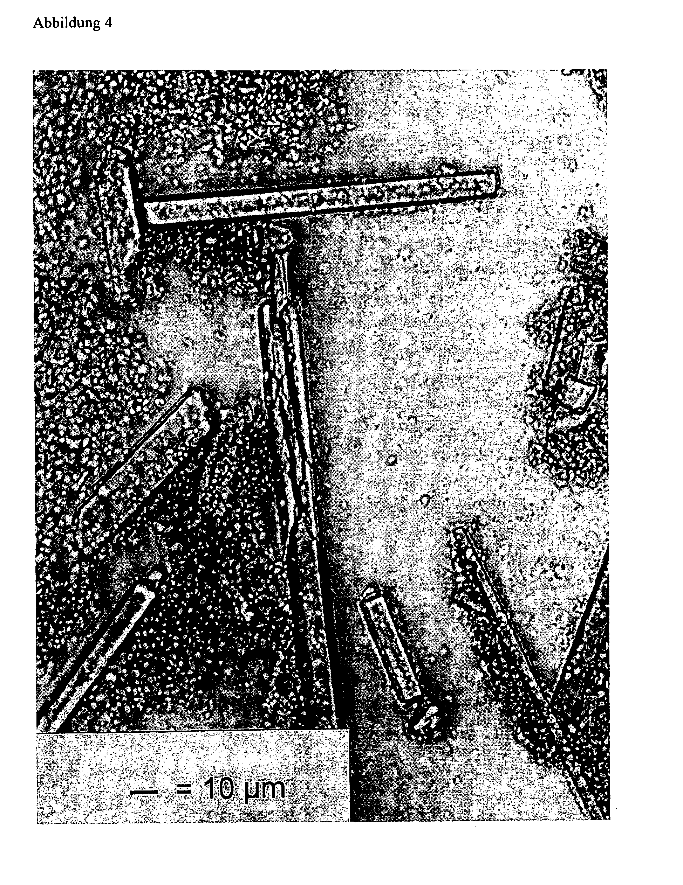

- Abbildung 4:: Lichtmikroskopische Untersuchung von Vergleichsbeispiel 1 nach oben beschriebener achtwöchiger Lagerung

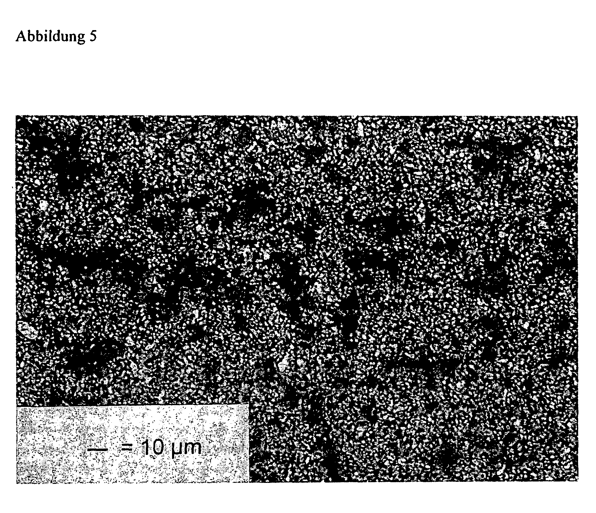

- Abbildung 5:: Lichtmikroskopische Untersuchung der erfindungsgemäßen Formulierung 16 nach oben beschriebener achtwöchiger Lagerung

### Beispiele für biologische Wirkung

### Knock-Down-Wirkung: Myzus persicae -Test

Zur Herstellung einer zweckmäßigen Anwendungslösung verdünnt man 1 Gewichtsteil formulierte Ware mit Wasser auf die gewünschte Konzentration.

Paprikapflanzen (*Capsicum annum*), die von allen Stadien der Grünen Pfirsichblattlaus (*Myzus persicae*) befallen sind, werden mit einer Anwendungslösung der gewünschten Konzentration gespritzt.

Unmittelbar nach Abtrocknen des Spritzbelages wird die Wirkung in % bestimmt. Dabei bedeutet 100 %, dass alle Blattläuse geschädigt wurden; 0 % bedeutet, dass keine Blattläuse geschädigt wurden.

Bei diesem Test zeigen z. B. die folgende Formulierungen überlegene Wirksamkeit gegenüber dem Stand der Technik: 15, 16.

**Tabelle 3**

| Pflanzenschädigende Insekten | | |
|---|---|---|
| **Myzus persicae - Test** | | |
| Wirkstoff/Produkt | Konzentration in g ai/ha | Abtötung in % nach 2^{h} |
| **Beispiel 16** | **1** + **0,43** | **80** |
| erfindungsgemäß | | |
| **Beispiel 15** | **1 + 0,27** | **90** |
| erfindungsgemäß | | |
| **Imidacloprid OD 200** | | |
| Stand der Technik | **1** | **60** |
| **ß-Cyfluthrin EC 100** | | |
| Stand der Technik | **0,43** | **50** |
| **ß-cyfluthrin SC 125** | | |
| Stand der Technik | **0,43** | **20** |
| **Deltamethrin EC 025** | | |
| Stand der Technik | **0,27** | **80** |
| **Deltamethrin SC 200** | | |
| Stand der Technik | **0,27** | **0** |

### Mortalität/Effektivität: Myzus persicae -Test

Zur Herstellung einer zweckmäßigen Anwendungslösung verdünnt man 1 Gewichtsteil formulierte Ware mit Wasser auf die gewünschte Konzentration.

Paprikapflanzen *(Capsicum annum*), die von allen Stadien der Grünen Pfirsichblattlaus (*Myzus persicae*) befallen sind, werden mit einer Anwendungslösung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgende Formulierungen überlegene Wirksamkeit gegenüber dem Stand der Technik: 15, 16

**Tabelle 4**

| Pflanzenschädigende Insekten | | |
|---|---|---|
| **Myzus persicae - Test** | | |
| Wirkstoff/Produkt | Konzentration in g ai/ha | Abtötung in % nach 1^{d} |
| **Beispiel 16** | **1 + 0,43** | **100** |
| erfindungsgemäß | | |
| **Beispiel 15** | **1 + 0,27** | **98** |
| erfindungsgemäß | | |
| **Imidacloprid OD 200** | | |
| Stand der Technik | **1** | **94** |
| **ß-Cyfluthrin EC 100** | | |
| Stand der Technik | **0,43** | **55** |
| **ß-Cyfluthrin SC 125** | | |
| Stand der Technik | **0,43** | **20** |
| **Deltamethrin EC 025** | | |
| Stand der Technik | **0,27** | **94** |
| **Deltamethrin SC 200** | | |
| Stand der Technik | **0,27** | **0** |

### Testbeschreibung: Penetrationsförderer auf der Ebene der Kutikula

Additive, die als Penetrationsförderer auf der Ebene der Kutikula wirken, seien nachfolgend als Akzelerator-Additive bezeichnet (vgl. Schönherr und Baur, 1994, Pesticide Science 42, 185-208). Akzelerator-Additive zeichnen sich dadurch aus, dass sie aus der wässrigen Spritzbrühe und/oder aus dem Spritzbelag in die Kutikula eindringen und dadurch die Stoffbeweglichkeit (Mobilität) von Wirkstoffen In der Kutikula erhöhen können. Andere Additive wie Polyethylenglykol wirken dagegen nur im Spritzbelag (über die Flüssigphase) oder wirken nur als Netzmittel wie z.B. Natriumdodecylsulfat.

In diesem Test wird der Einfluß von Additiven auf die Penetrationseigenschaften anderer Substanzen auf der Ebene der Kutikula bestimmt. Dabei wird die Mobilität einer Testsubstanz in der Kutikula ohne und mit einem Additiv über eine Desorptionsmethode gemessen. Die Methode ist detailliert in der Literatur veröffentlicht (Baur et al., 1997, Pesticide Science, 51, 131-152) und lediglich die Prinzipien und Abweichungen werden nachfolgend beschrieben.

Als Testsubstanz mit der Funktion eines Tracers wurde hier eine radioaktiv markierte schwache organische Säure ausgewählt. Als Pflanzenmaterial wurden die enzymatisch isolierten Blattkutikeln der Oberseite von Birnenblättern von Freilandbäumen verwendet. Die Kutikeln wurden in speziell angefertigte Diffusionszellen aus Edelstahl eingebaut. Der Tracer wurde in einem Citratpuffer bei pH 3 in gelöstem Zustand auf die ursprünglich dem Blattinneren zugewandten Seite appliziert. Diese Innenseite nimmt die kleine radioaktive Menge des Tracers in der nicht dissoziierten Säureform leicht auf. Anschließend wurde diese Innenseite abgedeckt und bei 100% Luftfeuchte gehalten. Die normalerweise luftexponierte, morphologische Aussenseite der Blattkutikula wurde dann mit einem Puffer (pH7), der Rezeptorlösung in Kontakt gebracht und die Desorption gestartet. Die penetrierte Säureform der Testsubstanz wird durch den Rezeptor dissoziiert und die Desorption erfolgt einer Kinetik erster Ordnung. Die Desorptionskonstante ist proportional der Mobilität des Tracers in der Kutikula.

Nach mindestens 2 Zeiten zur Bestimmung dieser Konstanten wird nun die Desorption mit einem Puffer fortgesetzt, der zusätzlich das zu testende Additiv enthält. Je nach Eigenschaft des Additives kommt es nun zur Sorption des Additives in der Kutikula und je nach Wirksamkeit als Weichmacher für die Kutikula erhöht sich die Mobilität des Tracers in der Kutikula. Dies äußert sich in einer erhöhten Desorptionskonstante und das Verhältnis der Steigungen mit Additiv zu dem ohne Additiv beschreibt den Effekt des Additives auf der Ebene der Kutikula als Penetrationsförderer zu wirken. Der Vergleich des mittleren Effektes verschiedener Additive gibt damit deren Wirksamkeit als Weichmacher der Kutikula zu agieren wieder.

## Patentansprüche

1. Zusammensetzung umfassend
- mindestens einen bei Raumtemperatur festen Wirkstoff aus der Reihe der Neonikotinoide,
- mindestens einen bei Raumtemperatur festen Wirkstoff aus der Reihe der Pyrethroide,
- mindestens einen Penetrationsförderer,
- mindestens ein Pflanzenöl,
- Cyclohexanon,
- mindestens ein nicht-ionisches Tensid und/oder mindestens ein anionisches Tensid und
- einen oder mehrere Zusatzstoffe aus den Gruppen der Emulgiermittel, der schaumhemmenden Mittel, der Konservierungsmittel, der Antioxydantien, der Spreitmittel, der Farbstoffe und/oder einen Verdicker.

2. Zusammensetzung gemäß Anspruch 1, wobei das Neonikotinoid ausgewählt ist aus der Gruppe Thiamethoxam, Clothianidin, Thiacloprid, Dinetofuran, Acetamiprid, Nitenpyram und Imidacloprid.

3. Zusammensetzung gemäß Anspruch 2, wobei das Neonikotinoid Imidacloprid ist.

4. Zusammensetzung gemäß einem der Ansprüche 1 bis 3, wobei das Pyrethroid ausgewählt ist aus beta-Cyfluthrin und Deltamethrin.

5. Zusammensetzung gemäß einem oder mehreren der Ansprüche 1 bis 4, wobei der Penetrationsförderer ein Alkanol-alkoxylat der Formel
R-O-(-AO)ₘR' (I)
in welcher
R für geradkettiges oder verzweigtes Alkyl mit 4 bis 20 Kohlenstoffatomen steht,
R' für H, Methyl, Ethyl, n-Propyl, i-Propyl, n-Butyl, i-Butyl, t-Butyl, n-Pentyl oder n-Hexyl steht,
AO für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
m für Zahlen von 2 bis 30 steht,
ist.

6. Zusammensetzung gemäß Anspruch 5, wobei der Penetrationsförderer ein Alkanol-Alkoxylat der Formel (I-a), (I-b), (I-c), (I-d), (I-e) oder (I-f) ist,
R-O-(-EO-)ₙ-R' (I-a)
R-O-(-EO-)ₚ-(-PO-)_{q}-R' (I-b)
R-O-(-PO-)ᵣ-(EO-)ₛ-R' (I-c)
R-O-(-EO-)ₚ-(-BO-)_{q}-R' (I-d)
R-O-(-BO-)ᵣ-(-EO-)ₛ-R' (I-e)
CH₃-(CH₂)ₜ-CH₂-O-(-CH₂-CH₂-O-)ᵤ-R' (I-f)
in welchen
R und R' die oben angegebenen Bedeutungen haben,
EO für -CH₂-CH₂-O- steht,
PO für 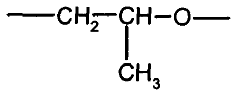 steht,
BO für 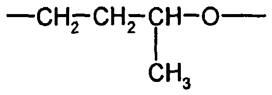 steht,
n für Zahlen von 2 bis 20 steht,
p, q, r und s für Zahlen von 1 bis 10 steht,
t für Zahlen von 8 bis 13 steht und
u für Zahlen von 6 bis 17 steht.

7. Zusammensetzung gemäß Anspruch 6, wobei der Penetrationsförderer ein Alkanol-Alkoxylat der Formel (I-c-1), (I-d-1) oder (I-f-1) ist
• 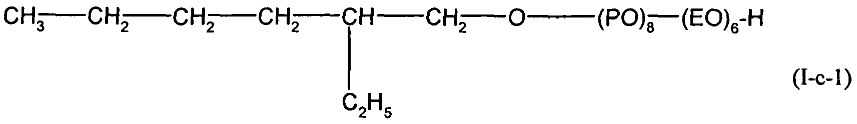 wobei die Zahlen 8 und 6 Durchschnittswerte darstellen,
•
CH₃-(CH₂)₁₀-O-(-EO-)₆-(-BO-)₂-CH₃ (I-d-1)
wobei die Zahlen 10, 6 und 2 Durchschnittswerte darstellen,
•
CH₃-(CH₂)_{10,5}-CH₂-O-(-CH₂-CH₂-O-)_{8,4}-R' (I-f-1)
wobei die Zahlen 10,5 und 8,4 Durchschnittswerte darstellen.

8. Zusammensetzung gemäß Anspruch 6, wobei der Penetrationsförderer ein Alkanol-Alkoxylat der Formel (I-f-1-1) ist
CH₃-(CH₂)_{10,5}-CH₂-O-(-CH₂-CH₂-O-)_{8,4}-H (I-f-1-1)
wobei die Zahlen 10,5 und 8,4 Durchschnittswerte darstellen.

9. Zusammensetzung gemäß einem oder mehreren der Ansprüche 1 bis 8, **dadurch gekennzeichnet, dass** sie
- an agrochemischen Wirkstoffen zwischen 5 und 40 Gew.-%,
- an Penetrationsförderer zwischen 5 und 55 Gew.-%,
- an Pflanzenöl zwischen 15 und 55 Gew.-%,
- an Cyclohexanon zwischen 5 und 20 Gew.-%,
- an Tensiden bzw. Dispergierhilfsmitteln zwischen 2,5 und 30 Gew.-% und
- an Zusatzstoffen zwischen 0,1 und 25 Gew.-%
enthält.

10. Zusammensetzung gemäß Anspruch 9, **dadurch gekennzeichnet, dass** sie
- an agrochemischen Wirkstoffen zwischen 10 und 37,5 Gew.-%,
- an Penetrationsförderer zwischen 10 und 35 Gew.-%,
- an Pflanzenöl zwischen 20 und 50 Gew.-%,
- an Cyclohexanon zwischen 7 und 16 Gew.-%,
- an Tensiden bzw. Dispergierhilfsmitteln zwischen 5 und 25 Gew.-% und
- an Zusatzstoffen zwischen 0,1 und 20 Gew.-%
enthält.

11. Verfahren zu Herstellung einer Zusammensetzung gemäß einem oder mehreren der Ansprüche 1 bis 10, **dadurch gekennzeichnet, dass** die Inhaltsstoffe miteinander vermischt und dann gemahlen werden, bis eine mittlere Teilchengröße von weniger als 10 µm erreicht ist.

12. Verfahren gemäß Anspruch 11, wobei der Mahlvorgang aus einer Grob- und einer Feinmahlung besteht und solange durchgeführt wird, bis 90% der Teilchen eine Teilchengröße von weniger als 6 µm aufweisen.

13. Verfahren gemäß Anspruch 11 oder 12, wobei zunächst Penetrationsförderer und Pflanzenöl vorgelegt werden und die übrigen Inhaltsstoffe dieser Mischung zugegeben werden.

14. Verfahren zur Bekämpfung von Schadinsekten, **dadurch gekennzeichnet, dass** eine Zusammensetzung gemäß einem oder mehreren der Ansprüche 1 bis 10 unverdünnt oder verdünnt in solcher Menge auf Insekten oder ihren Lebensraum appliziert wird, dass eine wirksame Menge der enthaltenen insektiziden Wirkstoffe auf die Insekten oder ihren Lebensraum wirkt.

## Claims

1. Composition comprising
- at least one room-temperature-solid active substance from the class of the neonicotinoids,
- at least one room-temperature-solid active substance from the class of the pyrethroids,
- at least one penetrant,
- at least one vegetable oil,
- cyclohexanone,
- at least one nonionic surfactant and/or at least one anionic surfactant, and
- one or more additives from the groups of the emulsifiers, foam inhibitors, preservatives, antioxidants, spreaders, colorants and/or a thickener.

2. Composition according to Claim 1, wherein the neonicotinoid is selected from the group of thiamethoxam, clothianidin, thiacloprid, dinetofuran, acetamiprid, nitenpyram and imidacloprid

3. Composition according to Claim 2, wherein the neonicotinoid is imidacloprid.

4. Composition according to any one of Claims 1 to 3, wherein the pyrethroid is selected from betacyfluthrin and deltamethrin.

5. Composition according to one or more of Claims 1 to 4, wherein the penetrant is an alkanol alkoxylate of the formula
R-O-(-AO)ₘR' (I)
in which
R is linear or branched alkyl having 4 to 20 carbon atoms,
R' is H, methyl, ethyl, n-propyl, isopropyl, n-butyl, isobutyl, tert-butyl, n-pentyl or n-hexyl,
AO is an ethylene oxide radical, a propylene oxide radical, a butylene oxide radical or mixtures of ethylene oxide and propylene oxide radicals or butylene oxide radicals, and
m is a number from 2 to 30.

6. Composition according to Claim 5, wherein the penetrant is an alkanol alkoxylate of the formula (I-a), (I-b), (I-c), (I-d), (I-e) or (I-f),
R-O-(-EO-)ₙ-R' (I-a)
R-O-(-EO-)ₚ-(-PO-)_{q}-R' (I-b)
R-O-(-PO-)ᵣ-(EO-)ₛ-R' (I-c)
R-O-(-EO-)ₚ-(-BO-)_{q}-R' (I-d)
R-O-(-BO-)ᵣ-(-EO-)ₛ-R' (I-e)
CH₃-(CH₂)ₜ-CH₂-O-(-CH₂-CH₂-O-)ᵤ-R' (I-f)
in which
R and R' are as defined above,
EO is -CH₂-CH₂-O-,
PO is 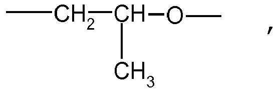
BO is 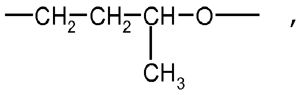
n is a number from 2 to 20,
p, q, r and s are numbers from 1 to 10,
t is a number from 8 to 13, and
u is a number from 6 to 17.

7. Composition according to Claim 6, wherein the penetrant is an alkanol alkoxylate of the formula (I-c-1), (I-d-1) or (I-f-1)
• 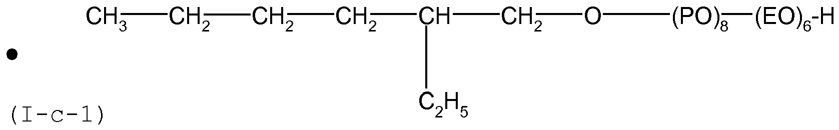 in which the numbers 8 and 6 are average values,
•
CH₃-(CH₂)₁₀-O-(-EO-)₆-(-BO-)₂-CH₃ (I-d-1)
in which the numbers 10, 6 and 2 are average values,
•
CH₃-(CH₂)_{10.5}-CH₂-O-(-CH₂-CH₂-O-)_{8.4}-R' (I-f-1)
in which the numbers 10.5 and 8.4 are average values.

8. Composition according to Claim 6, wherein the penetrant is an alkanol alkoxylate of the formula (I-f-1-1)
CH₃-(CH₂)_{10.5}-CH₂-O-(-CH₂-CH₂-O-)_{8.4}-H (I-f-1-1)
in which the numbers 10.5 and 8.4 are average values.

9. Composition according to one or more of Claims 1 to 8, **characterized in that** it comprises
- between 5% and 40% by weight of active agrochemicals,
- between 5% and 55% by weight of penetrant,
- between 15% and 55% by weight of vegetable oil,
- between 5% and 20% by weight of cyclohexanone,
- between 2.5% and 30% by weight of surfactants and/or dispersants, and
- between 0.1% and 25% by weight of additives.

10. Composition according to Claim 9, **characterized in that** it comprises
- between 10% and 37.5% by weight of active agrochemicals,
- between 10% and 35% by weight of penetrant,
- between 20% and 50% by weight of vegetable oil,
- between 7% and 16% by weight of cyclohexanone,
- between 5% and 25% by weight of surfactants and/or dispersants, and
- between 0.1% and 20% by weight of additives.

11. Process for producing a composition according to one or more of Claims 1 to 10, **characterized in that** the ingredients are mixed with one another and then ground until an average particle size of less than 10 µm is reached.

12. Process according to Claim 11, wherein the grinding operation is composed of a coarse grinding and a fine grinding is carried out until 90% of the particles have a size of less than 6 µm.

13. Process according to Claim 11 or 12, wherein penetrant and vegetable oil are introduced initially and the remaining ingredients of this mixture are added.

14. Method of controlling harmful insects, **characterized in that** a composition according to one or more of Claims 1 to 10 is applied neat or diluted to insects or their habitat in an amount such that an effective amount of the active insecticidal substances comprised acts on the insects or their habitat.

## Revendications

1. Composition, comprenant :
- au moins un agent actif solide à température ambiante du groupe des néonicotinoïdes,
- au moins un agent actif solide à température ambiante du groupe des pyréthroïdes,
- au moins un promoteur de pénétration,
- au moins une huile végétale,
- de la cyclohexanone,
- au moins un tensioactif non ionique et/ou au moins tensioactif anionique et
- un ou plusieurs additifs des groupes des émulsifiants, des agents antimousse, des conservateurs, des antioxydants, des agents d'étalement des colorants et/ou un épaississant.

2. Composition selon la revendication 1, dans laquelle le néonicotinoïde est choisi dans le groupe constitué par le thiaméthoxam, la clothianidine, le thiaclopride, le dinétofurane, l'acétamipride, le nitenpyram et l'imidaclopride.

3. Composition selon la revendication 2, dans laquelle le néonicotinoïde est l'imidaclopride.

4. Composition selon l'une quelconque des revendications 1 à 3, dans laquelle le pyréthroïde est choisi parmi la bêta-cyfluthrine et la deltaméthrine.

5. Composition selon une ou plusieurs des revendications 1 à 4, dans laquelle le promoteur de pénétration est un alcoxylate d'alcanol de formule
R-O-(-AO)ₘR' (I)
dans laquelle
R représente un alkyle linéaire ou ramifié de 4 à 20 atomes de carbone,
R' représente H, méthyle, éthyle, n-propyle, i-propyle, n-butyle, i-butyle, t-butyle, n-pentyle ou n-hexyle,
AO représente un radical oxyde d'éthylène, un radical oxyde de propylène, un radical oxyde de butylène ou des mélanges de radicaux oxyde d'éthylène et oxyde de propylène ou de radicaux oxyde de butylène, et
m représente des nombres de 2 à 30.

6. Composition selon la revendication 5, dans laquelle le promoteur de pénétration est un alcoxylate d'alcanol de formule (I-a), (I-b), (I-c), (I-d), (I-e) ou (I-f)
R-O-(-EO-)ₙ-R' (I-a)
R-O-(-EO-)ₚ-(-PO-)_{q}-R' (I-b)
R-O-(-PO-)ᵣ-(-EO-)ₛ-R' (I-c)
R-O-(-EO-)ₚ-(-BO-)_{q}-R' (I-d)
R-O-(-BO-)ᵣ-(-EO-)ₛ-R' (I-e)
CH₃-(CH₂)ₜ-CH₂-O-(-CH₂-CH₂-O-)ᵤ-R' (I-f)
dans lesquelles
R et R' ont les significations indiquées précédemment,
EO représente -CH₂-CH₂-O-,
PO représente 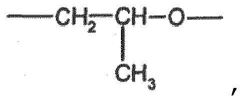
BO représente 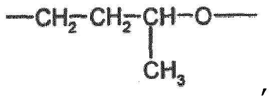
n représente des nombres de 2 à 20,
p, q, r et s représentent des nombres de 1 à 10,
t représente des nombres de 8 à 13 et
u représente des nombres de 6 à 17.

7. Composition selon la revendication 6, dans laquelle le promoteur de pénétration est un alcoxylate d'alcanol de formule (I-c-1), (I-d-1) ou (I-f-1)
• 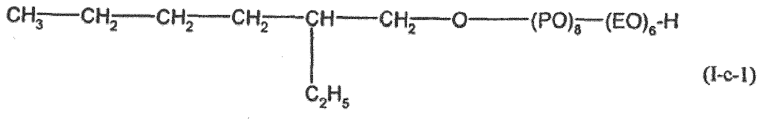 dans laquelle les nombres 8 et 6 sont des valeurs moyennes,
•
CH₃-(CH₂)₁₀-O-(-EO-)₆-(-BO-)₂-CH₃ (I-d-1)
dans laquelle les nombres 10, 6 et 2 sont des valeurs moyennes,
•
CH₃-(CH₂)_{10,5}-CH₂-O-(-CH₂-CH₂-O-)_{8,4}-R' (I-f-1)
dans les nombres 10,5 et 8,4 sont des valeurs moyennes.

8. Composition selon la revendication 6, dans laquelle le promoteur de pénétration est un alcoxylate d'alcanol de formule (I-f-1-1)
CH₃-(CH₂)_{10,5}-CH₂-O-(-CH₂-CH₂-O-)_{8,4}-H (I-f-1-1)
dans laquelle les nombres 10,5 et 8,4 sont des valeurs moyennes.

9. Composition selon une ou plusieurs des revendications 1 à 8, **caractérisée en ce qu'**elle contient
- des agents actifs agrochimiques entre 5 et 40 % en poids,
- des promoteurs de pénétration entre 5 et 55 % en poids,
- une huile végétale entre 15 et 55 % en poids,
- de la cyclohexanone entre 5 et 20 % en poids,
- des tensioactifs ou adjuvants de dispersion entre 2,5 et 30 % en poids, et
- des additifs entre 0,1 et 25 % en poids.

10. Composition selon la revendication 9, **caractérisée en ce qu'**elle contient :
- des agents actifs agrochimiques entre 10 et 37,5 % en poids,
- des promoteurs de pénétration entre 10 et 35 % en poids,
- une huile végétale entre 20 et 50 % en poids,
- de la cyclohexanone entre 7 et 16 % en poids,
- des tensioactifs ou adjuvants de dispersion entre 5 et 25 % en poids, et
- des additifs entre 0,1 et 20 % en poids.

11. Procédé de fabrication d'une composition selon une ou plusieurs des revendications 1 à 10, **caractérisé en ce que** les ingrédients sont mélangés les uns avec les autres, puis broyés jusqu'à une taille de particule moyenne de moins de 10 µm.

12. Procédé selon la revendication 11, dans lequel le processus de broyage est constitué d'un broyage grossier et d'un broyage fin, et est réalisé jusqu'à ce que 90 % des particules présentent une taille de particule de moins de 6 µm.

13. Procédé selon la revendication 11 ou 12, dans lequel le promoteur de pénétration et l'huile végétale sont tout d'abord chargés et les autres ingrédients sont ajoutés à ce mélange.

14. Procédé de lutte contre les insectes nuisibles, **caractérisé en ce qu'**une composition selon une ou plusieurs des revendications 1 à 10 sous forme non diluée ou diluée est appliquée sur les insectes ou leur habitat en une quantité telle qu'une quantité efficace des agents actifs insecticides contenus agisse sur les insectes ou leur habitat.
